# EUROPÄISCHE PATENTSCHRIFT

(11) **EP 2 185 061 B1**
(45) Veröffentlichungstag und Bekanntmachung des Hinweises auf die Patenterteilung: **10.10.2012**
(21) Anmeldenummer: 08785691.0
(22) Anmeldetag: 22.08.2008
(51) Int. Cl.: A61B 1/31, A61B 17/34, A61B 17/00

(54) **TROKARROHR, TROKAR, OBTURATOR BZW. REKTOSKOP FÜR DIE TRANSLUMINALE ENDOSKOPISCHE CHIRURGIE ÜBER NATÜRLICHE KÖRPERÖFFNUNGEN**
TROCAR TUBE, TROCAR, OBTURATOR, OR RECTOSCOPE FOR TRANSLUMINAL ENDOSCOPIC SURGERY VIA NATURAL BODY CAVITIES
FOURREAU DE TROCART, TROCART, OBTURATEUR ET RECTOSCOPE POUR CHIRURGIE ENDOSCOPIQUE TRANSLUMINALE PAR LES VOIES NATURELLES

(30) Priorität: 27.08.2007 DE 102007040358
(43) Veröffentlichungstag der Anmeldung: 19.05.2010
(73) Patentinhaber: Technische Universität München, 80333 München (DE)
(72) Erfinder: CAN, Salman, CH- 1700 Fribourg (CH); FIOLKA, Adam, 58642 Iserlohn (DE); SCHNEIDER, Armin, 80333 München (DE); FEUSSNER, Hubertus, 81671 München (DE)
(74) Vertreter: Vossius & Partner
(86) Internationale Anmeldenummer: PCT/EP2008/006936
(87) Internationale Veröffentlichungsnummer: WO 2009/027065

(56) Entgegenhaltungen:
- EP-A- 1 602 336
- WO-A-00/69350
- WO-A-2004/016184
- DE-A1- 4 129 237
- DE-A1- 4 312 147
- DE-A1- 10 100 756
- DE-A1- 19 935 725
- DE-U1- 20 119 222
- US-A1- 2005 251 190
- US-B1- 6 454 783

## Beschreibung

Die vorliegende Erfindung betrifft ein Trokarrohr, einen Trokar, einen Obturator und ein Rektoskop, insbesondere für die transluminale endoskopische Chirurgie über natürliche Körperöffnungen. Ein erfindungsgemäßes Trokarrohr bzw. ein entsprechender Trokar ist mit einem distalen Abschnitt zur Positionierung zumindest in dem Bereich oder außerhalb der Körperöffnung und einem proximalen Abschnitt zur Positionierung innerhalb des Menschen oder Tieres vorgesehen.

In der endoskopischen und laparoskopischen Chirurgie ist ein Punkt erreicht, an dem viele Verfahren minimal-invasiv durchgeführt werden können. Es lassen sich in Zukunft chirurgische Eingriffe im peritonealen Raum voraussichtilich auch gänzlich ohne Inzision der Bauchdecke ausführen. Der Zugang erfolgt dann über natürliche Körperöffnungen - also transgastrisch, transvaginal, transvesikal oder transcolonal. Der erste Bericht über ein derartiges Verfahren (hier: transgastrische Leberbiopsie) stammt aus dem Jahr 2004 [Kalloo AN, Singh VK, Jagannath SB, et al. Flexible transgastric peritoneoscopy: a novel approach to diagnostic and therapeutic interventions in the peritoneal cavity. Gastrointest Endosc 2004; 60; pp. 114-117]. Eine Übersicht über bisher durchgeführte Studien ist in einem kürzlich erschienenen Artikel aufgelistet [De la Fuente SG, De Maria EJ, Reynolds JD, et al. New Developments in Surgery. Arch Surg 2007; 142; pp. 295-297].

Führende Experten der amerikanischen Gesellschaft der gastrointestinalen Endoskopie (ASGE) und der Gesellschaft der amerikanischen gastrointestinalen und endoskopischen Chirurgen (SAGES) stimmten bei einem Treffen im Juli 2005 darin überein, dass dieses Verfahren unter dem Namen 'Natural Orifice Transluminal Endoscopic Surgery (NOTES)' für den Patienten bedeutende Verbesserungen, wie z.B. weniger Schmerz, schnellere Genesung und bessere Kosmetik bieten wird als gegenwärtige laparoskopische Methoden.

In der transluminalen Chirurgie werden die Instrumente bisher meist über eine künstlich geschaffene Öffnung in der Magenvorderwand, also transgastrisch, in die Bauchhöhle eingebracht. Obwohl sich derzeit der experimentelle Einsatz von NOTES weitestgehend auf Tiermodelle beschränkt berichten Rao et al. als erste Gruppe vom humanen Einsatz im Rahmen von Appendektomien [Rao GV, Reddy N. Transgastric appendectomy in humans. Oral presentation at the annual meeting of the Society of American Gastrointestinal and Endoscopic Surgeons (SAGES) 2006]. NOTES wurde in den USA im Jahr 2007 erstmalig zur Durchführung einer transgastrischen und transvaginalen Cholezystektomie eingesetzt. Als Nachteile des transgastrischen Zugangs werden insbesondere die für die Inspektion der / Intervention in den beiden oberen Quadranten des Abdomens erforderliche Retroflexion des Endoskops / der chirurgischen Instrumente, resultierend in einer Inversion der endoskopischen Sicht, sowie Komplikationen bezüglich eines sicheren Verschlusses der Magenwand am Ende der Intervention genannt. Pai et al. berichten erstmalig von einer transcolonal durchgeführten Cholezystektomie im Überlebensmodell [Pai RD, Fong DG, Bungda ME et al. Transcolonic endoscopic cholecysfectomy: a NOTES survival study in a porcine model. Gastrointest Endosc 2006; 64; pp. 428-434]. Fong et al. beschreiben in ihrer an sechs Schweinen durchgeführten Studie ebenfalls den Zugang in den Bauchraum über das Colon [Fong DG, Pai RD, Thompson CC. Transcolonic endoscopic abdominal exploration: a NOTES survival study in a porcine model. Gastrointest Endosc 2007; 65; pp. 312-318]. Allerdings verzeichnen beide Gruppen eine erhöhte bakterielle Kontamination des Bauchraums. Weitere transcolonale /transrectale Studien liegen mittlerweile unter anderem von McGee et al. [McGee MF, Marks JM, Chak A, et al. Feasibility of transrectal natural orifice transluminal endoscopic surgery (NOTES) in the porcine model. Poster presentation at the annual meeting of the Society of American Gastrointestinal and Endoscopic Surgeons (SAGES) 2007], Denk et al. [Denk PM, Whiteford MH, Swanstrom LL. TEM as a portal for NOTES. Poster presentation at the annual meeting of the Society of American and Gastrointestinal Endoscopic Surgeons (SAGES) 2007] und Horton et al. [Horton K, Hathaway P, Krehel G, et al. *New device for closure in transluminal surgery.* Oral presentation at the annual meeting of the Society of American Gastrointestinal and Endoscopic Surgeons (SAGES) 2007J vor.

Ausgehend von Schätzungen des 'US Center for Disease Control' leiden in den USA etwa 40 Millionen Menschen an Erkrankungen des Gastrointestinaltrakts, die mit direkten und indirekten Krankheitskosten von jährlich rund 100 Milliarden US$ von hoher sozio-ökonomischer Relevanz sind. Ein großer Anteil dieser Patienten könnte von NOTES profitieren, vorausgesetzt, die hypothetischen Vorteile von NOTES gegenüber der minimal-invasiven Chirurgie lassen sich kurz- bis mittelfristig auch in humanen klinischen Studien validieren. In der aktuellen Entwicklungsphase ist zunächst die Bereitstellung geeigneter Instrumente und Verfahren sowie eine richtungsweisende Klärung der noch offenen Fragestellungen hinsichtlich Zugangsweg, Infektionsrisiken, zuverlässigem Verschluss, geeigneter Nahtverfahren und potentieller Komplikationen von besonderer Bedeutung.

Für die transluminale Chirurgie wird in der Regel ein flexibles Operationsinstrument (modifiziertes Endoskop) über eine künstlich geschaffene Öffnung in der Magenvorderwand in die Bauchhöhle eingebracht. Mit diesem flexiblen Instrument können dann entsprechende Eingriffe im Bauchraum (bisher wurden Entfernungen der Gallenblase, des Blinddarms, Tubenligaturen usw. experimentell durchgeführt) vorgenommen werden. Nach Beendigung des Eingriffes und Zurückziehen des Gerätes kommt es darauf an, den Eintrittspunkt in das Abdomen in der Magenvorwand auch sicher zu verschließen.

Rektoskope sind im allgemeinen bekannt und beispielsweise in den Patentschriften EP 0 858 283 und DE 199 35 725 beschrieben. Aus der Druckschrift EP 1 100 368 ist ein starres Sigmoidoskop bekannt, aufweisend ein beseitigbares Spekulum, umfassend ein im wesentlichen hohles Rohr, das einen Innenraum definiert, ein Beobachtungsende und ein Einführende, wobei das Einführende des Spekulums ausgelegt ist für ein Einführen in den Analkanal eines Patienten, und eine Kopplungseinrichtung, die ausgelegt ist, um einer wieder verwendbaren Lichtquelle zu erlauben, mit dem Beobachtungsende verbunden zu werden und Licht durch das Spekulum in den Darm des Patienten zu projizieren.

Trokarsysteme sind in diversen Ausführungsformen vorbekannt. Sie bestehen in der Regel aus einem Trokarrohr sowie einem innerhalb des Trokarrohrs bewegbaren Obturator, welcher an seinem distalen Ende mit einer scharfen, vorteilhaft pyramidenförmig ausgestalteten Spitze oder Schneide zur Durchdringung von Gewebe ausgestattet ist. Das Trokarrohr kann starr oder flexibel ausgebildet sein.

Die Offenlegungsschrift US 2005/0251190 offenbart eine Trokarvorrichtung zur Schaffung einer Zugangsöffnung in eine Körperhöhle. Die Druckschrift US 2007/0088277 beschreibt eine chirurgische Zugangsöffnung bestehend aus einem dichten Trokargehäuse mit mindestens einer Dichtung, einem Trokarrohr mit einem Lumen, das von der mindestens einen Dichtung abgedichtet wird und einem lösbaren Anschlusssystem zwischen Trokarrohr und Trokargehäuse.

Dichtungsvorrichtungen sind unter anderem auch aus den Schriften US 4,943,280, US 4,655,752, US 4,978,341 und EP 0 567 141 bekannt. Letztgenannte betrifft ein Ventilsystem, das das Einführen chirurgischer Instrumente in den Körper eines Patienten insbesondere über eine Trokarvorrichtung erlaubt. Ein gasdichter Abschluss ist für Instrumente unterschiedlicher Größe gewährleistet.

In der EP 0 630 213 wird ein Trokaraufbau mit einziehbarer Spitze beschrieben, die aus einer Schneidposition in eine abgeschirmte Position zurückgezogen werden kann. Ein weiterer Sicherheitstrokar ist aus der Patentschrift EP 0 604 197 bekannt. Die Anmeldung EP 0 535 974 beansprucht eine Trokar-Vorrichtung bestehend aus einem Trokarrohr, das ein inneres Lumen definiert und am distalen und proximalen Ende eine Öffnung besitzt, so dass ein länglich ausgebildetes Instrument mit kleinerem Durchmesser innerhalb dieses Trokarrohrs bewegt werden kann, dadurch gekennzeichnet, dass das Trokarrohr aus einer elastisch ausgebildeten Hülse gefertigt ist und über eine inhärente Rückstellkraft verfügt, so dass das Trokarrohr nach Anwendung einer Biegekraft wieder in seine geradlinige Ausrichtung zurückkehrt.

In der EP 0 538 359 wird ein durch eine Öffnung in den Körper einführbares Rohr mit Biegemechanismus beschrieben, das mit einem Manipulator für die Bewegung von wenigstens einem Paar von Steuerdrähten versehen ist. Die Patentschrift DE 40 02 235 offenbart eine Führungsvorrichtung für das perkutane Einbringen eines Endoskopes mit einem Einführungsteil und einem distalen Winkelteil, gekennzeichnet durch einen Trokar bestehend aus einer Außenhülse und einem Obturator oder Griffteil, der herausnehmbar in dieser Außenhülse eingesetzt ist und einem Führungsrohr, welches in die Außenhülse anstelle des Obturators einsetzbar ist, um einen Einbringungskanal zu bilden. Dabei ist das Führungsrohr aus einem Metallrohr gebildet und im vorderen Ende im Querschnitt gesehen kurvenförmig abgerundet.

Die Patentschrift DE 43 12 147 zeigt einen Trokar mit einem Obturator mit einer Bohrspitze zum Durchbohren von Körpergewebe; einer Kanüle zum Bilden eines Durchlasses in dem von der Bohrspitze durchbohrten Gewebe, um ein Endoskop oder chirurgisches Werkzeug in eine Körperhöhle einführen zu können, wobei die Kanüle aus einer flexiblen Röhre mit einem offenen proximalen Ende besteht; und einem Gehäuse, das an dem offenen proximalen Ende der Kanüle angeordnet ist und ein Abdichtventil zum abgedichteten Führen des Obturators aufweist, dadurch gekennzeichnet, dass das Gehäuse aus einem weichen Material gefertigt ist. Die Kanüle ist aus einer Schraubenfederwicklungsröhre gebildet.

In der Offenlegungsschrift DE 41 29 237 ist eine Trokarhülse zum Durchführen von Hilfsinstrumenten dargestellt, dadurch gekennzeichnet, dass sie zumindest auf einem Teil ihrer Länge derart biegsam ausgebildet ist, dass sich ihre Form durch elastisches Biegen, im übrigen aber knickfest etwaigen Krümmungen von starren Hilfsinstrumenten anpasst. Die Trokarhülse kann mehrere Längskanäle aufweisen.

Die Druckschrift DE 28 20 239 zeigt eine ähnliche Vorrichtung. Die Offenlegungsschrift US 2004/0044350 beansprucht eine gelenkige Einführschleuse zur Schaffung eines Zugangs zu einer Körperhöhle bestehend aus einem Schaft mit einem proximalen und einem distalen Ende und einem zentralen Lumen, durch das chirurgische Instrumente in die Körperhöhle eingeführt werden können. Ein Teil des Schaftes beinhaltet eine Anzahl an Gelenkgliedern, so dass dieser Teil des Schaftes über mindestens einen durch mindestens ein Gelenkglied verlaufenden Zugdraht in eine gekrümmte Form gebracht werden kann. Ferner wird ein im zentralen Lumen positionierbarer, ggf. flexibler Obturator offenbart, der im beweglichen Teil des Schaftes eine erste Formgebung vermittelt.

Im Hinblick auf Vorrichtungen für die transluminale Chirurgie sei exemplarisch auf folgende Publikationen hingewiesen: Die Anmeddeschrift EP 1 602 336 beansprucht ein Verfahren und eine Vorrichtung zur Schaffung eines transmuralen Zugangs zum Inneren einer Körperhöhle bestehend aus einer flexiblen, multi-luminalen Außenhülse mit einem distalen Ende und einem zentralen Lumen zur Aufnahme eines Endoskops und einem Außenlumen zur Bestückung mit einem Befestigungsmechanismus zur Sicherung des distalen Endes der multi-luminalen Außenhülse an der Organwand. Gemäß dem zugehörigen europäischen Recherchenbericht wird hier unter anderem die Patentschrift US 6,030,365 als Entgegenhaltung aufgeführt. Dort ist ein Verfahren und eine Vorrichtung zur Schaffung eines sterilen, chirurgischen Zugangs zu inneren Körperteilen über eine natürliche Öffnung einer innen liegenden Körperhöhle beschrieben. Die Vorrichtung wird vorteilhaft in das Rektum eines Patienten eingeführt. Neben einem Hauptzugangskanal ist in einer möglichen Ausgestaltungsform ein parallel dazu verlaufender, konzentrischer Absaugkanal vorgesehen.

Transgastrische Zugänge zur Bauchhöhle und entsprechende Verfahren sind unter anderem speziell in den Anmeldeschriften US 2004/0260245, US 2005/0148818 und US 2006/0237022 aufgeführt.

Schließlich wird auf die Anmeldung WO 2004/016184 verwiesen, in der ein kurzer Trokar mit einer starken Krümmung offenbart wird, der beispielsweise bei einer Thorakoskopie eingesetzt wird. Insbesondere beschreibt die WO 2004/016184 ein gebogenes Trokarrohr mit einem Biegungswinkel von 60° bis 70°, das bei Thorakoskopien besonders vorteilhaft ist.

Der Erfindung liegt die Aufgabe zugrunde, ein verbessertes Trokarrohr, einen verbesserten Trokar, einen verbesserten Obturator bzw. ein verbessertes Rektoskop, insbesondere zur Verwendung in der transluminalen endoskopischen Chirurgie über natürliche Körperöffnungen eines Menschen oder Tieres, bereitzustellen.

Diese Aufgabe wird anhand eines Trokarrohrs gemäß dem unabhängigen Patentanspruch 1 gelöst. Vorteilhafte Weiterbildungen sind Gegenstand der abhängigen Ansprüche.

Die Erfindung betrifft ein Trokarrohr zur Verwendung in der transluminalen endoskopischen Chirurgie ûber das Rektum eines Menschen, mit einem distalen Abschnitt zur Positionierung zumindest in dem Bereich oder außerhalb des Rektums, und einem proximalen Abschnitt zur Positionierung innerhalb des Menschen, wobei zumindest der proximale Abschnitt eine im Wesentlichen formfeste bzw. verbiegefeste Krümmung aufweist. Formfest bzw. verbiegefest heißt, dass das Trokarrohr im Gebrauch im Wesentlichen seine Form, vor allem die Krümmung, bei bestimmungsgemäßem Gebrauch beibehält. Die Erfindung umfasst zusätzlich einen Obturator für das Trokarrohr bzw. einen erfindungsgemäßen Trokar, wobei zumindest ein proximaler Abschnitt eines Schaftes des Obturators aus flexiblem und/oder elastischem Material hergestellt ist, so dass er sich beim Einschieben in das Trokarrohr der Krümmung des proximalen Abschnitts des Trokarrohrs anpassen kann.

Der Trokar ist ein Instrument, mit dessen Hilfe in der Chirurgie scharf oder stumpf eine Öffnung in eine Körperhöhle (z. B. Bauchraum, Brustraum) geschaffen und durch ein Trokarrohr bzw. Tubus offen gehalten wird. Es handelt sich üblicherweise um einen Obturator bzw. Stift, der in dem Trokarrohr mit einem Innendurchmesser von z.B. 0,5-12 mm sitzt und dessen Spitze die Öffnung des Trokarrohrs verschließt. Der Trokar wird z. B. durch die Bauchdecke in den Bauchraum eingeführt. Der Operateur hat dann nach Herausziehen des Obturators aus dem Trokarrohr die Möglichkeit, mit einer Optik (Endoskop) durch das Trokarrohr in den Bauchraum zu schauen oder mit Greif-, Schneide- und anderen Instrumenten innerhalb des Bauchraumes minimal-invasiv zu operieren. Dies kann durch einen mit dem Trokarrohr distal verbundenen Trokarkopf geschehen, der einen gasdichten Port für die genannten Instrumente aufweist. Moderne Trokare sind entweder aus chirurgischem Stahl oder Kunststoff, werden als einmal oder mehrfach verwendbare Instrumente hergestellt. Die Spitze des Obturators ist an das Trokarrohr angepasst und hat vorzugsweise eine oder mehrere scharfe Schneidkanten für scharfe Präparation des Zugangsweges oder hat eine stumpfe, konische Spitze zur stumpfen Präparation. Die Trokarrohre speziell für die Inspektion von Körper- bzw. Gelenkhöhlen (Laparoskopie, Thorakoskopie, Arthroskopie) können einen Ventilmechanismus bzw. einen Anschluss zum Insufflieren (Einblasen) von CO₂ oder Spülflüssigkeiten oder andere Zusatzausstattungen aufweisen.

Gemäß dem Stand der Technik ist das Trokarrohr flexibel und der Obturator in seiner ungeraden Form inflexibel. Demgegenüber lässt sich das formfeste und zumindest teilweise gekrümmte erfindungsgemäße Trokarrohr besser führen und setzen und ein entsprechender Obturator besser führen, um die Perforation des Abdomens, insbesondere der anterioren Rektalwand zielgenau und präzise durchführen zu können. Vorzugweise ist das Trokarrohr lang genug, um einen Zugang in die Bauchhöhle zu ermöglichen und ggf. die zuvor genannten beispielhaften Operationen durchführen zu können.

Bevorzugt ist das gesamte Trokarrohr im Wesentlichen verbiegefest. Vorzugsweise ist es aus einem leicht sterilisierbaren Material, wie z.B. aus einem geeigneten Kunststoff, Metall usw. hergestellt. Kunststoff ist aus Kosten- und Gewichtsgründen bevorzugt. Das erfindungsgemäße Trokarrohr ist vorzugsweise einzeln oder im Set mit den weiteren beschriebenen und beanspruchten Elementen als Wegwerfartikel oder nach einer Sterilisierung verpackt.

Bei dem bevorzugten erfindungsgemäßen Trokarrohr ist die Krümmung des proximalen Abschnitts im Wesentlichen konstant. Es kann aber auch mit zunehmendem und/oder abnehmenden Radius oder mit mehreren Radii zum freien Ende hin ausgeformt sein. Die Form ist gemäß den anatomischen Voraussetzungen und anderen operationsbedingten Umständen unterschiedlich vorgebbar. Ein bevorzugter Krümmungsradius einer Mittelachse des proximalen Abschnitts des Trokarrohrs beträgt etwa 45-75 mm, vorzugsweise etwa 50-70 mm, weiter bevorzugt etwa 55-65 mm und weiter bevorzugt etwa 60 mm.

Ein erfindungsgemäßes Trokarrohr hat vorzugsweise über einen zumindest wesentlichen Teil der Gesamtlänge einen Innendurchmesser von etwa 2-22 mm, bevorzugt von etwa 10-20 mm, weiter bevorzugt von etwa 14-18 mm, weiter bevorzugt von etwa 15-17 mm und weiter bevorzugt von etwa 16 mm.

Weiter bevorzugt weist ein Trokarrohr über einen zumindest wesentlichen Teil der Gesamtlänge eine Wandungsstärke von etwa 0,2-2 mm, bevorzugt von etwa 0,8-1,2 mm, weiter bevorzugt von etwa 0,9-1,1 mm, weiter bevorzugt von etwa 1 mm auf. Besonders bevorzugt ist das gesamte Trokarrohr mit dieser Wandstärke versehen, ausgenommen hiervon ist nur die später erwähnte Befestigungsvorrichtung für einen Trokarkopf am distalen Ende und eine Konifizierung am proximalen Ende.

Das erfindungsgemäße Trokarrohr hat eine Gesamtlänge von etwa 230-400 mm, bevorzugt von etwa 280-370 mm, weiter bevorzugt von etwa 300-350 mm, weiter bevorzugt von etwa 315-335 mm und weiter bevorzugt von etwa 325 mm. Die Länge des Trokars ist so ausgelegt, dass er bis in die Bauchhöhle eingeführt werden kann und im Körper frei manipulierbar ist. Der Trokar bietet eine Befestigungsmöglichkeit, damit er an einem Halter oder Manipulator angebracht werden kann. Wegen der kurvenförmigen Konfiguration des Rektosigmoids hat der gebogene Vorderteil des Trokars eine Ski-ähnliche Form. Dadurch kann das Rektum einfacher durchstochen werden. Zusätzlich kann infolge der gebogenen Form des Trokars, das Endoskop im Situs vorpositioniert werden.

Bevorzugt beträgt die wirksame Länge eines geraden Abschnitts des Trokarrohrs etwa 180-300 mm, bevorzugt etwa 200-280 mm, weiter bevorzugt etwa 220-260 mm, weiter bevorzugt etwa 232-252 mm, weiter bevorzugt etwa 238-247 mm und weiter bevorzugt etwa 242 mm. Zur Gesamtlänge kommen dann noch die Länge des gekrümmten Bereichs und die Länge eines Bereichs zur Verbindung an den Trokarkopf.

Erfindungsgemäß beträgt die absolute Gesamtauslenkung des Trokarrohrs im Bereich der Krümmung des proximalen Abschnitts zu einer Seite etwa 15-18 mm, bevorzugt etwa 16-17 mm, weiter bevorzugt etwa 16,5 mm. Dabei wird der Abstand parallel zur benachbarten Außenwand des Trokarrohrs in seinem geraden Abschnitt bis zum äußersten Punkt des proximalen Abschnitts, bevorzugt an dessen freiem Ende, gemessen. Alternativ kann auch der maximale Winkel zwischen einer Längsachse des nicht gekrümmten bzw. geraden Abschnitts des Trokarrohrs und der Längsachse bzw. ihrer Tangente des am weitesten gekrümmten proximalen Abschnitts gemessen werden. Dieser maximale Winkel beträgt etwa 20-70°, bevorzugt etwa 30-60°, weiter bevorzugt etwa 35-55°, weiter bevorzugt etwa 40-50° und weiter bevorzugt etwa 45°. Im letzten Fall heißt dies bei einer bevorzugten gleichmäßigen Krümmung, dass der gekrümmte Abschnitt 1/8 eines vollen Kreisbogens beschreibt. Es wäre jedoch auch 1/3 oder ¼ eines vollen Kreisbogens für bestimmte Zwecke denkbar.

Die zuvor genannten Maße sind besonders für die vorliegende Verwendung über den rektosigmoidalen Übergang in das Abdomen geeignet.

Vorzugsweise ist ein freies Ende des proximalen Abschnitts konifiziert. Bevorzugt hat zumindest der proximale Abschnitt, vorzugsweise das gesamte Trokarrohr, einen im Wesentlichen gleichbleibenden Querschnitt, der vorzugsweise oval oder kreisförmig bzw. rund ist. Ein kreisförmiger Querschnitt ist weniger aufwendig, dafür bietet ein ovaler Querschnitt den Vorteil, dass ein später beschriebener erfindungsgemäßer Obturator verdrehfest geführt werden kann, wenn z.B. die Form und Positionierung seiner scharfen Spitze eine besondere Rolle spielt. Für ein später eingeführtes Endoskop stellt ein ovaler Querschnitt außerdem noch ein wenig mehr Bewegungsspielraum dar.

Weiter erfindungsgemäß weist das Trokarrohr eine Einrichtung zum flüssig-, vorzugsweise gasdichten Verbinden mit einem Trokarkopf auf. Die Verbindungseinrichtung hat bevorzugt eine radiale Ausbauchung zum formschlüssig elastischen Hintergreifen der Ausbauchung durch eine entsprechende Vorrichtung am Trokarkopf.

Das Trokarrohr hat weiterhin bevorzugt außen Markierungen zum Ablesen der radialen Orientierung und/oder seiner eingeführten Länge. Dadurch wird dem Operateur eine Hilfe für die Positionierung und ggf. Weiterführung des Trokarrohrs gegeben.

Das Trokarrohr kann außerdem erfindungsgemäß noch mindestens einen Arbeitskanal, der vorzugsweise außen an der Außenwand des Trokarrohrs angeordnet ist, aufweisen. Durch einen derartigen Arbeitskanal können ebenfalls Hilfsmittel, Geräte usw. zur Stelle der Perforation und/oder in den Bauchraum zielgenau von außen eingebracht werden.

Vorzugsweise weist ein erfindungsgemäßer Trokar ein Trokarrohr und einen Trokarkopf auf. Beides kann einstückig, vereinzelt oder zusammen in einem Kit bereitgestellt werden bzw. steril verpackt sein.

Vorzugsweise sind das Trokarrohr und der Trokarkopf flüssig- und bevorzugt gasdicht unter Zwischenlage einer Dichtung und/oder einer formschlüssigen Verbindung miteinander verbindbar oder bereits einstückig ausgeführt. Auch diese Dichtung kann in dem genannten Kit vorhanden sein.

Erfindungsgemäß können das Trokarrohr und der Trokarkopf lösbar miteinander verbunden werden. Dies würde einen modularen Aufbau zum individuellen Zusammenstellen der am besten geeigneten Komponenten bzw. auch das individuelle Ausbilden eines der Komponenten als Wegwerfartikel ermöglichen. Der Trokarkopf ist in der Regel aufwendiger und daher könnte im letzteren Falle nur das Trokarrohr als Wegwerfartikel aus einem geeigneten Kunststoff angeboten werden.

Der Trokarkopf ist bevorzugt mit mindestens einem gasdichten Ventil und/oder mindestens einem Einlass und/oder Auslass für Gas(e) und/oder Flüssigkeit(en), z.B. zur Insufflation von Gas oder dem Ablassen von Taurolidin-Lösung versehen.

Weiterhin weist der Trokar vorzugsweise eine Einrichtung zum Befestigen des Trokars, vorzugsweise an einem Halter oder Manipulator, auf.

Der schon genannte erfindungsgemäße Obturator weist eine Spitze auf, die derart konifiziert ist, dass sie der Konifizierung des freien Endes des proximalen Abschnitts des Obturators zum besseren Perforieren der Darmwand im Wesentlichen entspricht. Alternativ oder zusätzlich können der erfindungsgemäße Trokar bzw. das Trokarrohr auch mit anderen chirurgischen Werkzeugen verwendet bzw. in einen Kit verpackt werden.

Damit das Gewebe, das die Körperöffnung umgibt (beispielsweise der Dickdarm), während des Einführens des Trokars durch den Obturator nicht unbeabsichtigt verletzt wird und/oder die vorzugsweise scharfe und vorzugsweise konifizierte bzw. kegelförmige Spitze des Obturators nicht beschädigt wird, ist eine kontrollierte Einführung des Obturators bevorzugt. Die kegelförmige Spitze des Obturators ist vorzugsweise beim Einführen des Trokars in das Trokarrohr zurückgezogen positionierbar bzw. versenkbar, so dass die Spitze des Obturators nicht nach vorne über das Trokarrohr hinausragt. D.h. der Obturator befindet sich in einer zurückgezogenen Position. Nachdem der Trokar zu der Einstichstelle geführt wurde, kann die Perforation der Darmwand (vorzugsweise unter optischer Kontrolle) durch Herausschieben bzw. Herausdrücken des Obturators erfolgen. Durch das Herausschieben des Obturators steht die Obturatorspitze über das proximale Ende des Trokarrohrs hervor, so dass die Spitze in Eingriff mit dem umgebenden Gewebe gebracht werden kann, d.h. der Obturator ist in einer ausgefahrenen Position.

Vorzugsweise weist der Trokar am distalen Ende eine Rückhalteeinrichtung auf, um den Obturator in der zurückgezogenen Position zu halten bzw. in die zurückgezogene Position vorzuspannen. Die Rückhalteeinrichtung weist vorzugsweise mindestens ein federndes Element, beispielsweise eine oder mehrere Druckfedern auf, die den Obturator in die zurückgezogene Position vorspannen. Wird eine dem federnden Element entgegenwirkende Kraft ausgeübt, so wird der Obturator entgegen der Vorspannung vorgeschoben (d.h. in die proximale Richtung), so dass die Spitze aus dem Trokarrohr herausragt, damit das umgebende Gewebe, beispielsweise die Darmwand, durchstochen werden kann. Nach dem Loslassen des Obturators wird der Obturator mit der kegelförmigen Spitze mittels Rückhalteeinrichtung in die Ausgangsposition, d.h. die zurückgezogene Position, zurückgezogen. Vorzugsweise weist die Rückhalteeinrichtung Anschläge auf, zwischen denen der Obturator von einer zurückgezogenen Position zu einer ausgefahrenen Position über eine vorbestimmte Länge hin und her bewegt werden kann. Ein erster Anschlag kann so ausgelegt sein, dass die Spitze des Obturators auf der Höhe des proximalen Endes des Trokars oder tiefer innerhalb des Trokars liegt, d.h. der Obturator ist in einer zurückgezogenen sicheren Position. Ein zweiter Anschlag kann so ausgelegt sein, dass sich die gesamte Spitze des Obturators über das proximale Ende des Trokars erstreckt. Vorzugsweise ist der zweite Anschlag so ausgelegt, dass die Obturatorspitze bündig an dem konifizierten proximalen Ende des Trokarrohrs liegt.

Die Spitze des Obturators hat einen maximalen Außendurchmesser von etwa 2-21 mm, bevorzugt von etwa 11-19 mm, weiter bevorzugt von etwa 13-17 mm, weiter bevorzugt von etwa 14-16 mm und weiter bevorzugt von etwa 15 mm. Weiter bevorzugt hat ein hinter der Spitze des Obturators angeordneter Schaft des Obturators einen maximalen Außendurchmesser von etwa 6-19 mm, bevorzugt von etwa 8-17 mm, weiter bevorzugt von etwa 10-15 mm, weiter bevorzugt von etwa 11-14 mm und weiter bevorzugt von etwa 12 mm.

Vorzugsweise hat der Trokar, also das Trokarrohr und der Trokarkopf und die Spitze des vollständig eingeführten Obturators eine Gesamtlänge von etwa 280-420 mm, bevorzugt von etwa 300-400 mm, bevorzugt von etwa 320-380 mm, weiter bevorzugt von etwa 340-360 mm und weiter bevorzugt von etwa 350 mm.

Die Erfindung betrifft ebenfalls ein dem zuvor genannten Trokarrohr angepasstes Rektoskop. Bei den weiteren Ausführungsbeispielen handelt es sich nicht um einen Teil der Erfindung. Sie dienen Lediglich dem Verständnis der Erfindung. Das Rektoskop weist weist einen Port für den Trokar auf und der Port ist derart ausgebildet, dass er bei eingeführtem Trokar eine Kippbewegung des Trokars zum Einstechen der Darmwand ermöglicht. Mit einem Rektoskop wird üblicherweise eine Mastdarmspiegelung (Rektoskopie) durchgeführt. Diese dient der Untersuchung des Enddarmes und meistens auch der letzten Zentimeter des Rektums.

Das Rektoskop hat einen Rohrschaft, dessen proximales Ende eine Abschrägung aufweist. Diese Abschrägung und der Port sind so ausgerichtet, dass sie die Kippbewegung des Trokars ermöglichen bzw. weiter unterstützen oder es erst ermöglichen, dass eine Obturatorspitze die entsprechend freigelegte Darmwand perforieren kann. Eine vorzugsweise in der Nähe der Perforationsstelle eingebrachte von außen einsehbare Optik kann dann die Obturatorspitze und die Perforation beobachtbar machen. Vorzugsweise hat die Abschrägung einen Winkel zur Mittelachse des Rohrschaftes von etwa 45°-80°, bevorzugt von etwa 60°-80°, weiter bevorzugt von etwa 65°-75° und weiter bevorzugt von etwa 70°.

Das Rektoskop weist einen Rohrschaft und mindestens zwei unterschiedliche Kappen vorzugsweise mit Schnellverschluß zum distalen gasdichten Verschließen des Rohrschafts auf. Dabei kann eine der Kappen mit dem Port für den Trokar und die andere Kappe für das Einführen von weiteren Operationsinstrumenten vorgesehen sein. Alternativ kann auch nur eine Kappe mit Ports für alle zu verwendenden Gerätschaften und den Trokar vorgesehen sein.

Das Rektoskop hat eine Gesamtlänge von etwa 150-250 mm, bevorzugt von etwa 170-230 mm, weiter bevorzugt von etwa 180-220 mm, weiter bevorzugt von etwa 190-210 mm und weiter bevorzugt von etwa 200 mm.

Wie schon erwähnt, betrifft die Erfindung auch ein Set oder mehrere Sets mit mindestens zwei Elementen eines Trokarrohrs, eines Trokars, eines Obturators und/oder eines Rektoskops. Denkbar wäre ein Set mit allen diesen Elementen und weitere Sets für die nachfolgende Benutzung nur mit einem Trokarrohr und einem Obturator und evtl. mit einem Trokarkopf.

Die zuvor und nachfolgend beschriebenen und beanspruchten Elemente können für die transluminale endoskopische Chirurgie und/oder Diagnostik über das Rektum verwendet werden.

Die Erfindung kann ebenfalls in einem Verfahren zur Vorbereitung einer Behandlung, zur Behandlung bzw. Diagnostik in der transluminalen endoskopischen Chirurgie über das Rektum eines Menschen oder Tieres verwendet werden. Ein derartiges Verfahren umfasst die Schritte des Bereitstellens eines Trokarrohrs, eines Trokars, eines Obturators, und/oder eines Rektoskops und ggf. die entsprechende Bedienung.

Am Beispiel einer Intervention wird das Einsatzfeld der neuen Erfindung erklärt. Das intraabdominale Lumen des rektosigmoidalen Übergangs ist mittels eines speziellen Rektoskops leicht zugänglich. Auf der Höhe des rektosigmoidalen Übergangs wird eine Tabaksbeutelnaht gesetzt. Unter Sicht wird anschließend der endoskopische Trokar mit eingesetztem Obturator in das Rektoskop eingeführt und die anteriore Rektalwand präzise durchstochen, um einen Zugang in die Bauchhöhle zu ermöglichen. Nachdem der Obturator entfernt ist, kann das eigentliche flexible Operationsinstrument in die Bauchhöhle eingeführt werden. Nach der Beendigung der intraabdominalen Prozedur wird das flexible Operationsinstrument und der endoskopische Trokar entfernt und die Tabaksbeutelnaht durch das Rektoskop verschlossen.

Vorzugsweise wird die Perforation der Darmwand unter Ultraschallkontrolle durchgeführt um beispielsweise eine Verletzung der Blase beim Einstechen des Trokars zu verhindern. Vor diesem heiklen Einstechvorgang mit dem Obturator wird vorzugsweise nach der Einführung des Rektoskops und nach dem Nähen der Tabaksbeuteinaht der vorgesehene Einstichpunkt mit einer Punktierungsnadel, die einen Nadeldraht aufweist, gesetzt. Danach kann der Trokar mit dem Obturator über den Nadeldraht bis zum Einstichpunkt vorgeschoben und die Darmwand anschließend an der vorgesehenen Stelle perforiert werden.

Dieser bevorzugt kontrollierte Vorgang erfolgt vorzugsweise unter Sicht einer endoskopischen Ultraschallsonde, die ebenfalls in das Rektoskop eingeführt wird. Dadurch kann die Verletzung zusätzlicher umliegender Organe wie z.B. einer Dünndarmschlinge verhindert werden. Vorzugsweise wird ein(e) endoskopische(r) Ultraschallkopf bzw. Ultraschallsonde durch einen Instrumentenkanal des Rektoskops eingeführt. Durch einen weiteren Instrumentenkanal kann eine Punktierungsnadel mit Hilfe von einem speziellen, vorzugsweise proximal verjüngten und vorzugsweise gebogenen Führungsinstrument eingeführt werden, wodurch die Darmwand an der richtigen Stelle durch die Tabaksbeutelnaht durchstochen werden kann. Das Führungsinstrument wird anschließend entfernt. Über den bzw. entlang des eingeführten und vorzugsweise verbleibenden Nadeldraht(s) kann der Obturator, vorzugsweise zusammen mit dem Trokar, vorgeschoben werden.

Die Punktierungsnadel bzw. der Nadeldraht hat vorzugsweise einen Durchmesser von ca. 0,5-3 mm. Das führungsinstrument kann aus unterschiedlichen Materialien hergestellt werden, z. B. Kunststoff, rostfreiem Stahl, Formgedächtnismaterialien usw. Das Führungsinstrument hat vorne (proximal) vorzugsweise einen kleinen Durchmesser, so zum Beispiel von 1-4 mm. Zudem hat das Führungselement am proximalen Ende vorzugsweise einen gebogenen Abschnitt, der aus Festigkeits- und Stabilitätsgründen vorzugsweise eine kurze Länge, vorzugsweise von ca. 10-40 mm aufweist. Der hintere (distale) Teil des Führungsinstruments hat vorzugsweise einen größeren Durchmesser, beispielsweise von ca. 3-7mm, vorzugsweise von 5 mm. Gemäß bevorzugten Ausführensformen hat das Führungsinstrument zumindest am hinteren Ende eine Wandstärke von min. 1 mm. Mit Hilfe eines optionalen distalen Griffs kann das Führungsinstrument zudem einfacher eingeführt und bedient werden. Vorzugsweise weist das Führungsinstrument Markierungen entlang der Längsrichtung auf, so dass die Eindringtiefe des Instruments einfach überprüft werden kann.

Als weitere Unterstützung für den Einstechvorgang kann der vorzugsweise flexible oder halbstarre Obturator einen zentralen Einführungs- bzw. Führungskanal für die Punktierungsnadel aufweisen. Durch diesen Führungskanal kann mit der Punktierungsnadel unter optischer Sicht ein weiter verbesserter, kontrollierter Einstechvorgang bzw. Perforationsvorgang durchgeführt werden. Dieser Führungskanal ist vorzugsweise vorne (proximal) auf den Durchmesser der Nadel angepasst und hat hinten (distal) vorzugsweise einen Durchmesser von ca. 2-4 mm.

Das vorliegende verfahren ermöglicht einen sicheren, sterilen, sigmoidalen Zugang zum Peritonealraum. Unter Verwendung von Taurolidin-Lösung - einer seit 1975 in der septischen Abdominalchirurgie und seit 1980 in der Traumatologie erfolgreich als chirurgische Spüllösung zur Behandlung von Infektionen angewandte Substanz mit antibakterieller und fungizider Wirkung bei fehlenden toxischen Kurz- oder Langzeiteffekten - konnten im Rahmen erster Untersuchungen am Schweinemodell keine kontaminationsbedingten Komplikationen beobachtet werden. Es wird vorzugsweise zunächst ein Schutzfluid im Bauchraum in an sich bekannter Weise, vorzugsweise über eine Verres-Nadel, eingebracht. Über diese Nadel wird daraufhin vorzugsweise ca. 1 I Taurolin-Lösung (Taurolin, Boehringer Ingelheim, Ingelheim, Deutschland) und vorzugsweise ca. 2.5 I Ringer-Lösung eingebracht. Im Versuch wurden dann Schweine in die sogenannte Anti-Trendelenburg Position gebracht (Kopf 30° nach oben). Da sich das injiziierte Fluid vor allem im kleinen Becken sammelt, werden die beweglichen und gasaufweisenden Organe usw. im Becken vom Beckenboden und dem Rektosigmoid entfernt. Auf diese Weise wird der später zu perforierende Bereich in vorteilhafter Weise freigelegt. Es ergibt sich also bei der richtigen Lagerung eines Patienten eine Freilegung des rektosigmoidalen Übergangs, so dass der zu perforierende Bereich weitgehend von Organen usw. frei bleibt und ein für den Patienten sichereres Vorgehen ermöglicht wird.

Ein großer Vorteil der Erfindung ist es, mit Hilfe eines geeigneten Gerätesystems einen sterilen, sicheren Zugang über den rektosigmoidalen Übergang in das Abdomen zu gewährleisten und zudem einen veriässlichen Verschluss der Eintrittsstelle nach Beendigung des Eingriffes möglich zu machen. Vor allem die Manövrierbarkeit des Trokars bzw. des Trokarrohrs zum gezielten Positionieren des Obturators ist wesentlich vorteilhafter als im Stand der Technik möglich.

Ein weiterer Vorteil der Erfindung ist es, ein chirurgisches Instrument anzugeben, das einen sterilen Zugang über den Rektosigrnoidalbereich in die Bauchhöhle vermittelt und am Ende der Intervention einen sicheren Verschluss der Eintrittsöffnung erlaubt.

Vorteilhafte Ausführungsformen der Erfindung werden beispielhaft in den Figuren dargestellt. Es zeigen:
- Fig. 1: eine perspektivische und schematische Darstellung eines erfindungsgemäßen Trokars mit eingeführtem Obturator,
- Fig. 2: einen Querschnitt durch einen erfindungsgemäßen Trokar mit eingeführtem Obturator,
- Fig. 3: einen Querschnitt durch ein Rektoskops,
- Fig. 4: eine perspektivische und schematische Darstellung eines Rektoskops mit eingeführtem erfindungsgemäßen endoskopischem Trokar und Obturator,
- Fig. 5: einen Querschnitt durch einen Trokar mit eingeführtem Obturator gemäß einer weiteren erfindungsgemäßen Ausführungsform und
- Fig. 6: einen Querschnitt durch ein Rektoskop mit eingeführtem Führungsinstrument und eingeführter Ultraschallsonde.

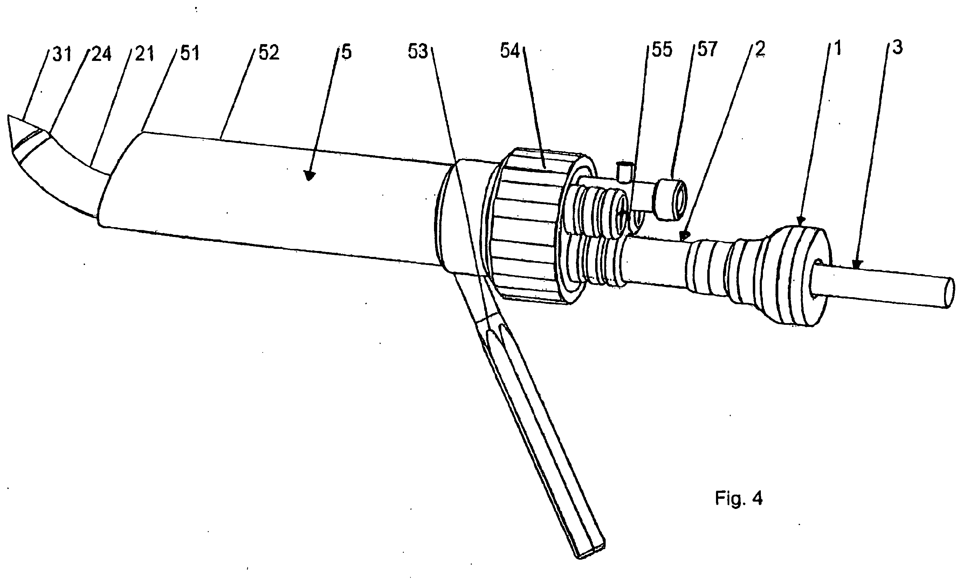

Fig. 4 zeigt am besten die gemeinsame Anordnung oder Wirkungsweise der erfindungsgemäßen Elemente Trokar, Obturator und Rektoskop. Ein Rektoskop 5 beherbergt ein Trokarrohr 2 und einen Obturator 3. Diese Elemente sind weiter in den anderen Figuren gezeigt. Ein ebenfalls bevorzugt verwendeter Trokarkopf ist in Fig. 4 nicht gezeigt.

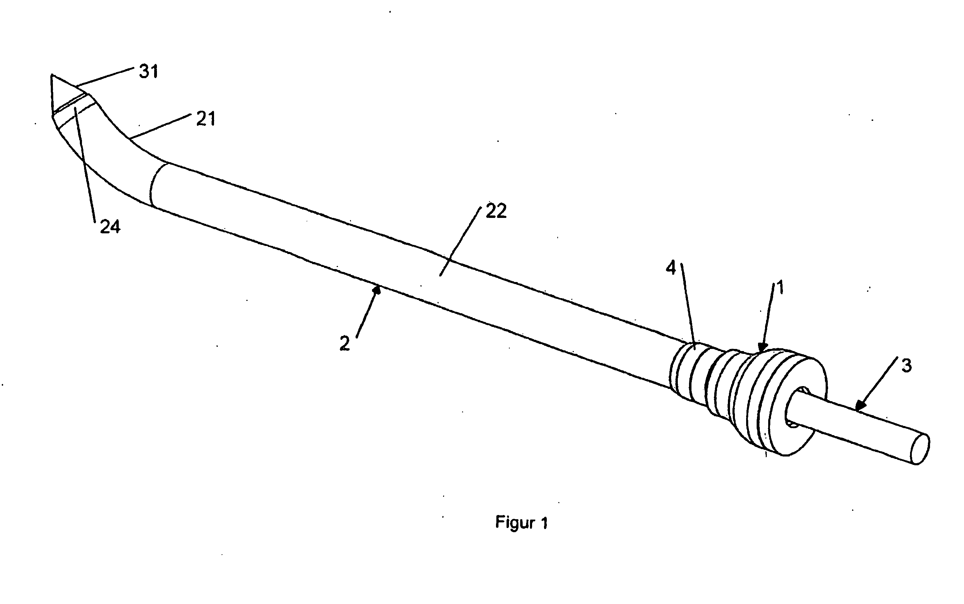

Fig. 1 zeigt dabei vor allem eine Anordnung aus dem erfindungsgemäßen Trokarrohr 2 mit einem daran angeordnetem Trokarkopf 1. Eine Dichtung 4 ist ebenfalls gezeigt, die vorzugsweise zum gasdichten Abdichten zwischen dem Trokarrohr 2 und dem Trokarkopf 1 angeordnet ist und auch eine elastische Zwischenlage zur besseren Lösbarkeit dieser Elemente darstellen kann. Der Obturator 3 mit einer Spitze 31 ist ebenfalls dargestellt und erstreckt sich durch das Trokarrohr 2 und den Trokarkopf 1. Das Trokarrohr 2 hat einen gekrümmten Abschnitt 21 und kann mindestens einen geraden bzw. ungekrümmten Abschnitt 22 aufweisen.

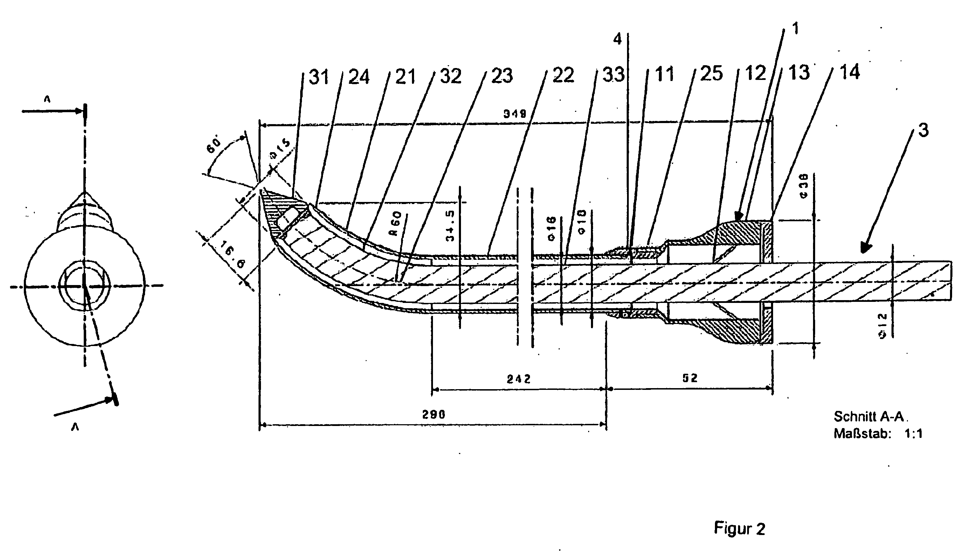

Fig. 2 zeigt in einem Querschnitt das Zusammenwirken dieser Elemente. Das Trokarrohr mit dem gekrümmten und dem geraden Abschnitt 21, 22 ist an seinem distalen Ende über die Dichtung 4 mit dem Trokarkopf 1 verbunden. Der Trokarkopf 1 hat einen Körper 13 und ist in einer bevorzugten Ausführungsform mit einer Ausbauchung am Trokarrohr 2 gezeigt, die von einer entsprechenden Aussparung am Trokarkopf 1 in an sich bekannter Weise umgriffen wird. Die Dichtung ist in einem Abschnitt zwischen dem Trokarkopf 1 und dem Trokarrohr 2 außerhalb dieser Verbindung zwischen diesen Elementen in einem Bereich gezeigt, in dem sich ein Ansatz 11 am Trokarkopf 1 weiter über das Trokarrohr 2 erstreckt. Andere bekannte Verbindungen, wie vor allem Click-, Bajonett-, Schraub-Verbindungen usw., sind ebenfalls geeignet, insbesondere wenn sie gasdicht ausführbar sind. Durch die dargestellte Ausführungsform ist die Anordnung zusätzlich drehgesichert.

Der Trokarkopf 1 weist außerdem ein Ventil 12 auf, das einen Schaft 33 des Obturators 3 gasdicht abdichtet. Weiterhin ist ein Deckel 14 vorgesehen, der einen Durchlass auch der größeren Spitze 31 wie das Ventil 12 gestattet, jedoch vor allem als mechanischer Schutz vorgesehen ist.

Das Trokarrohr 2 zeigt einen proximal zu dem Trokarkopf gelegenen, geraden Abschnitt 22, an dem sich ein gekrümmter Abschnitt 21 anschließt. Alternativ können auch - je nach anatomischen Gegebenheiten oder weiteren Besonderheiten - die Abschnitte in anderer Reihenfolge vorliegen oder mehrere gerade und/oder gekrümmte Abschnitte 22, 21 vorhanden sein. In der dargestellten Ausführungsform jedoch sieht man eine bevorzugte Anordnung, die anatomisch und ergonomisch vorteilhaft ist, vor allem für die zuvor genannte Verwendung.

Der gekrümmte Abschnitt 21 weist eine seitliche Auslenkung auf, die in der dargestellten Bemaßung zusammen mit dem Außendurchmesser (insgesamt 34,5 mm) dargestellt ist. Zieht man den am meisten bevorzugten Außendurchmesser von 18 mm davon ab, erhält man eine maximale Auslenkung von 16,5 mm.

Der in Fig. 2 dargestellte Radius R60 bezieht sich auf die Mittellinie 23 des Trokarrohrs 2.

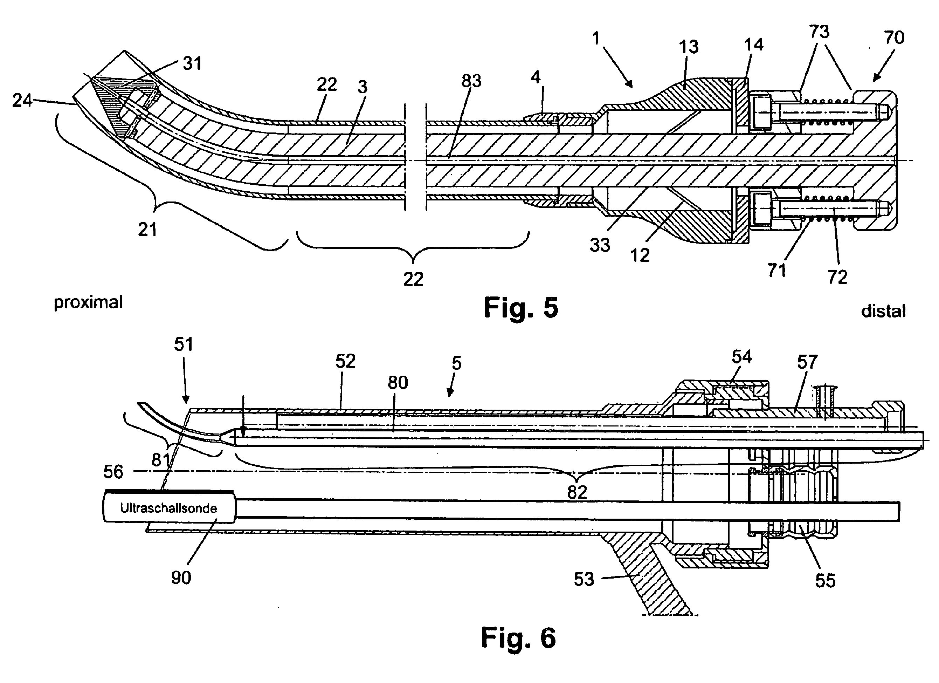

Am vorderen (proximalen) Ende des Trokarrohrs 2 erkennt man das konifizierte Ende 24, dessen Konifizierung sich vorteilhafterweise bei ausgefahrenem Obturator an der Spitze 31 des Obturators 3 fortsetzt. Diese vorzugsweise scharfe Spitze 31 ist in geeigneter Weise, vorzugsweise durch einen Stift, an dem Schaft 32, 33 angebracht. Damit beispielsweise der Dickdarm während des Einführens des Trokars durch die Spitze 31 des Obturators nicht unbeabsichtigt verletzt wird und/oder die vorzugsweise scharfe und vorzugsweise konifizierte bzw. kegelförmige Spitze 31 des Obturators nicht beschädigt wird, kann eine Rückhalteeinrichtung 70 am distalen (hinteren) Ende des Trokars vorgesehen sein (siehe Figur 5). Figur 5 zeigt eine der Figur 2 sehr ähnliche Ausführungsform, jedoch mit zusätzlicher Rückhalteeinrichtung 70. Die kegelförmige Spitze 31 des Obturators ist vorzugsweise beim Einführen des Trokars in das Trokarrohr zurückgezogen positionierbar bzw. versenkbar, sodass die Spitze des Obturators nicht nach vorne über das Trokarrohr hinausragt. Dies wird durch die Rückhalteeinrichtung 70 erreicht. Die Rückhalteeinrichtung 70 weist vorzugsweise mindestens einen Federmechanismus 71, 72 mit mindestens einem federnden Element 71 auf (beispielsweise mit einer oder mehreren Druckfedern 71), das den Obturator in die zurückgezogene Position vorspannt. In der in Figur 5 dargestellten Ausführungsform werden die Federn 71 auf den Schäften 72 (beispielsweise mit einer oder mehreren Zylinderschrauben) geführt. Nachdem der Trokar zu der gewünschten Einstichstelle geführt wurde, kann die Perforation der Darmwand durch Herausschieben bzw. Herausdrücken des Obturators erfolgen. Hierzu wird eine dem federnden Element entgegenwirkende Kraft ausgeübt, so dass der Obturator entgegen der Vorspannung vorgeschoben (d.h. in die proximale Richtung) wird und die Spitze 31 aus dem Trokarrohr herausragt, damit die Darmwand durchstochen bzw. perforiert werden kann. Wird keine Kraft mehr ausgeübt, wird der Obturator mit der kegelförmigen Spitze 31 in die Ausgangsposition zurückgezogen. Vorzugsweise weist die Rückhalteeinrichtung Anschläge 73 auf, zwischen denen der Obturator von einer zurückgezogenen Position zu einer ausgefahrenen Position hin und her bewegt werden kann. Durch geeignete Wahl der Anschläge kann die Länge, über die der Obturator verschoben werden kann, definiert werden. Ein erster Anschlag ist beispielsweise so ausgelegt, dass die Spitze des Obturators auf der Höhe des proximalen Endes des Trokars oder tiefer im Trokarrohr liegt, d.h. der Obturator ist in einer zurückgezogenen Position. Dies kann beispielsweise durch eine geeignete Länge der Schäfte 72 erreicht werden. Ein zweiter Anschlag 73 kann so ausgelegt sein, dass die Spitze 31 des Obturators aus dem proximalen Ende des Trokars hervorsteht. Vorzugsweise ist dieser Anschlag so ausgelegt, dass die Obturatorspitze bündig an dem konifizierten proximalen Ende 24 des Trokarrohrs liegt.

Zumindest ein Abschnitt 32 des Schaftes des Obturators 3 ist flexibel ausgebildet, um sich dem gekrümmten Abschnitt 21 des Trokarrohrs 2 anpassen zu können. Zusätzlich kann er noch elastisch ausgebildet sein, jedoch ist auch eine plastisch verformbare Variante denkbar. Er sollte jedoch nicht oder wenig kompressibel sein, damit die Spitze 31 möglichst abhängig von der Bewegung des entgegengesetzten Endes bewegbar ist. An dem flexiblen Abschnitt 32 ist ein weiterer Abschnitt 33 angeordnet, der damit nicht unbedingt einstückig ausgebildet sein muss und auch starr oder jedenfalls nicht elastisch sein kann. In einer bevorzugten Ausführungsform ist der gesamte Schaft 32, 33 jedoch aus einem flexiblen Material ausgebildet.

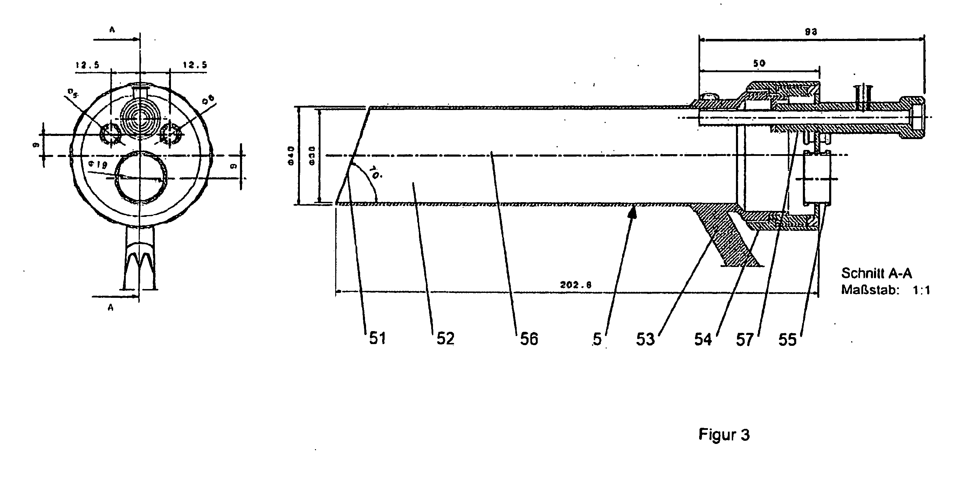

Figuren 3 und 4 zeigen eine Ausführungsform eines Rektoskops 5, welches besonders für den oben beschriebenen Trokar (Trokarrohr 2 mit eingeführtem Obturator 3 und Trokarkopf 1 in Fig. 4) geeignet ist. Das Rektoskop 5 besteht aus einem Rohrschaft 52 mit einer Längsachse 56 und einer Kappe 54. Beide Elemente sind vorzugsweise durch einen Schnellverschluss miteinander gasdicht verbunden. Ein Griff 53 unterstützt die Handhabung bzw. Positionierung durch den Operateur. Ein Port 55 ist vorgesehen, um das Trokarrohr 2 aufzunehmen bzw. gasdicht abzudichten. Besonders vorteilhafterweise für die Verwendung gestattet der Port 55 ein Verschwenken bzw. Verkippen des Trokars bzw. Trokarrohrs 2 zum Bewegen der in Fig. 4 nochmals gezeigten Spitze 31 und Perforieren der Darmwand. Es ist auch die Abschrägung 51 am proximalen Ende des Rektoskops 5, genauer des Rohrschafts 52, deutlich erkennbar. Diese Abschrägung 51 verbessert die Exposition des zu perforierenden Darmanteils und ist vorzugsweise so zum Port 55 angeordnet, dass der weiter durch die Abschrägung 51 freigelegte Teil des Rohrschafts dem Port 55 diametral gegenüber der Längsachse 56 gegenüberliegt (vgl. Fig. 3 und 6).

Weiterhin ist in der Fig. 3 ein verkürzt dargestellter optischer Kanal 57 gezeigt. Es ist vor allem in Figuren 3, 4 und 6 erkennbar, dass der Port 55, der optische Kanal 57 und ggf. weitere Einlässe in oder an der Kappe 54 angebracht sind, die vorzugsweise gasdicht sind. Für die Durchführung der Operation durch das Rektoskop 5 sind vorzugsweise zwei unterschiedliche Kappen notwendig. Durch die erste Kappe, bestehend aus drei Einführungskanälen für Operationsinstrumente, werden die Tabaksbeutelnaht und für die Operation notwendige mikrochirurgische Interventionen durchgeführt. Die zweite Kappe besteht ebenfalls aus drei Einführungskanälen. Diese Kappe wird nach dem Setzen der Tabaksbeutelnaht angebracht und ermöglicht die Einführung des endoskopischen Trokars. Die Eingangskanäle für Instrumente sind mit Ventilen versehen, um die Gasdichtigkeit zu gewährleisten. Die Optik wird durch einen speziellen Kanal eingeführt und ermöglicht je nach Intervention die Exposition des Operationsfeldes. Durch einen Schnellverschluss wird die Optik zusätzlich gesichert und abgedichtet.

Gemäß einer Ausführungsform wird die Perforation der Darmwand mittels Obturator unter Ultraschallkontrolle durchgeführt, um beispielsweise eine Verletzung der Blase beim Einstechen des Trokars zu verhindern. Vor diesem heiklen Einstechvorgang mit dem Obturator wird vorzugsweise nach der Einführung des Rektoskops 5 und nach dem Nähen der Tabaksbeutelnaht der gewünschte Einstichpunkt mit einer Punktierungsnadel bzw. einem Punktierdraht gesetzt. Die Punktiernadel bzw. der Punktierdraht wird vorzugsweise durch ein Drahtführungsinstrument bzw. Führungsinstrument 80, das in das Rektoskop eingeführt werden kann, geführt (siehe Figur 6). Vorzugsweise wird das Führungsinstrument 80 über einen der oberen Instrumentenkanäle 57 in das Rektoskop eingeführt. Das Führungsinstrument 80 weist vorzugsweise einen proximal verjüngt und vorzugsweise gebogenen Abschnitt 81 zur Führung der Punktierungsnadel bzw. des Punktierdrahts an die richtige Stelle durch die Tabaksbeutelnaht in der Darmwand auf. Dieser Vorgang geschieht vorzugsweise unter Ultraschallkontrolle. Hierzu kann beispielsweise ein endoskopischer Ultraschallkopf oder eine Ultraschallsonde 90 durch den unteren großen Einführungskanal 55 der Rektoskopkappe eingeführt werden. Das Führungsinstrument 80 wird anschließend entfernt, wobei der Draht im Rektoskop verbleibt. Über den eingeführten Nadeldraht kann anschließend der Trokar mit dem Obturator vorgeschoben werden, d.h., der Nadeldraht dient als Führung für den Obturator. Der Trokar mit dem Obturator 3 wird über den Nadeldraht bis zum Einstichpunkt vorgeschoben, sodass die Darmwand anschließend an der richtigen Stelle perforiert werden kann. Durch diesen bevorzugten kontrollierten Vorgang unter Sicht mittels der endoskopischen Ultraschallsonde 90 kann die Verletzung zusätzlicher umliegender Organe wie z.B. einer Dünndarmschlinge verhindert werden.

Eine Punktierungsnadel hat vorzugsweise einen Durchmesser von ca. 0,5-3 mm. Das Führungsinstrument 80 kann aus unterschiedlichen Materialien, wie z. B. Kunststoff, rostfreiem Stahl, Formgedächtnismaterialien usw. hergestellt werden. Das Führungsinstrument 80 hat vorne 81 vorzugsweise einen kleinen Durchmesser, so zum Beispiel von 1-4 mm. Der vordere Abschnitt 81 ist vorzugsweise gebogen und weist aus Festigkeits- bzw. Stabilitätsgründen vorzugsweise eine kurze Länge, vorzugsweise von ca. 10-40 mm auf. Der hintere Teil 82 des Instruments 80 hat vorzugsweise einen größeren Durchmesser, beispielsweise von ca. 3-7 mm, vorzugsweise 5 mm. Gemäß bevorzugten Ausführungsformen hat das Führungsinstrument 80 zumindest am hinteren Ende eine Wandstärke von min. 1 mm. Mit Hilfe eines optionalen distalen Griffs (nicht dargestellt) kann das Instrument einfacher eingeführt und bedient werden. Vorzugsweise weist das Instrument Markierungen in der Längsrichtung auf, so dass die Eindringtiefe des Instruments 80 einfach überprüft werden kann.

Als weitere Unterstützung für den Einstechvorgang kann der vorzugsweise flexible oder halbstarre Obturator einen zentralen Einführungs- bzw. Führungskanal 83 (siehe Figur 5) für die Punktierungsnadel aufweisen. Durch diesen Führungskanal 83 kann mit der Punktierungsnadel unter optischer Sicht ein weiter verbesserter, kontrollierter Einstechvorgang durchgeführt werden. Dieser Führungskanal 83 ist vorzugsweise vorne auf den Durchmesser der Nadel angepasst und hat hinten vorzugsweise einen Durchmesser von ca. 2-4 mm.

Durch nicht dargestellte äußere Markierungen am Trokarrohr kann die radiale Orientierung des Trokars abgelesen werden. Die Markierungen werden benutzt, um den gekrümmten Abschnitt bzw. das gebogene Vorderteil des Rohrs auszurichten. Durch zusätzliche Markierungen kann auch die aktuell im Körper sich befindende Trokarlänge abgelesen werden.

Der Schutzumfang der Erfindung wird in den Ansprüchen definiert.

## Patentansprüche

1. Trokarrohr zur Verwendung in der transluminalen endoskopischen Chirurgie über das Rekturn eines Menschen, mit einem distalen Abschnitt (22) zur Positionierung zumindest in dem Bereich oder außerhalb des Rektums und einem proximalen Abschnitt (21) zur Positionierung innerhalb des Menschen
, wobei zumindest der proximale Abschnitt (21) zumindest teilweise eine im Wesentlichen formfeste Krümmung aufweist,
wobei das Trokarrohr (2) einen geraden Abschnitt aufweist und das Trokarrohr (2) eine Gesamtlänge von etwa 230-400 mm hat,
**dadurch gekennzeichnet, dass** der proximale Abschnitt (21):
i) eine absolute Gesamtauslenkung zu einer Seite von etwa 15-18 mm aufweist und
ii) der maximale Winkel zwischen einer Längsachse des geraden Abschnitts des Trokarrohrs und der Längsachse des am weitesten gekrümmten proximalen Abschnitts etwa 20-60° beträgt.

2. Trokarrohr zur Verwendung in der transluminalen endoskopischen Chirurgie über das Rektum eines Menschen, mit einem distalen Abschnitt (22) zur Positionierung zumindest in dem Bereich oder außerhalb des Rektums und einem proximalen Abschnitt (21) zur Positionierung innerhalb des Menschen , wobei zumindest der proximale Abschnitt (21) zumindest teilweise eine im Wesentlichen formfeste Krümmung aufweist,
wobei das Trokarrohr (2) eine Gesamtlänge von etwa 230-400 mm hat, **dadurch gekennzeichnet, dass**
der distale Abschnitt (22) des Trokarrohrs (2) gerade ist und eine Länge von 180-300 mm aufweist, und
der maximale Winkel zwischen einer Längsachse des distalen geraden Abschnitts (22) des Trokarrohrs und der Längsachse des am weitesten gekrümmten proximalen Abschnitts (21) etwa 20-50° beträgt.

3. Trokarrohr nach Anspruch 1 oder 2, wobei das gesamte Trokarrohr (2) im Wesentlichen formfest ist.

4. Trokarrohr nach mindestens einem der vorstehenden Ansprüche, wobei die Krümmung des proximalen Abschnitts (21) im Wesentlichen konstant ist und einen Krümmungsradius einer Mittelachse des proximalen Abschnitts (21) von etwa 45-75 mm, vorzugsweise von etwa 50-70 mm, weiter bevorzugt von etwa 55-65 mm und weiter bevorzugt von etwa 60 mm aufweist.

5. Trokarrohr nach mindestens einem der vorstehenden Ansprüche, wobei das Trokarrohr (2) über einen zumindest wesentlichen Teil der Gesamtlänge einen Innendurchmesser von etwa 2-22 mm, bevorzugt von etwa 10-20 mm, weiter bevorzugt von etwa 14-18 mm, weiter bevorzugt von etwa 15-17 mm und weiter bevorzugt von etwa 16 mm aufweist.

6. Trokarrohr nach mindestens einem der vorstehenden Ansprüche, wobei das Trokarrohr (2) über einen zumindest wesentlichen Teil der Gesamtlänge eine Wandungsstärke von etwa 0,2-2 mm, bevorzugt von etwa 0,8-1,2 mm, weiter bevorzugt von etwa 0,9-1,1 mm, weiter bevorzugt von etwa 1 mm aufweist.

7. Trokarrohr nach Anspruch 1 oder einem der Ansprüche 3 bis 6, sofern abhängig von Anspruch 1, wobei die wirksame Länge des geraden Abschnitts des Trokarrohrs etwa 180-300 mm, bevorzugt etwa 200-280 mm, weiter bevorzugt etwa 220-260 mm, weiter bevorzugt etwa 232-252 mm, weiter bevorzugt etwa 238-247 mm und weiter bevorzugt etwa 242 mm beträgt.

8. Trokarrohr nach mindestens einem der vorstehenden Ansprüche, wobei das Trokarrohr (2) außen Markierungen zum Ablesen der radialen Orientierung und/oder der eingeführten Länge aufweist.

9. Trokarrohr nach mindestens einem der vorstehenden Ansprüche, wobei das Trokarrohr (2) noch vorzugsweise außen mindestens einen Arbeitskanal aufweist.

10. Trokar mit einem Trokarrohr (2) nach mindestens einem der vorstehenden Ansprüche und einem Trokarkopf (1), wobei das Trokarrohr (2) und der Trokarkopf (1) flüssig- bzw. gasdicht, vorzugsweise unter Zwischenlage einer Dichtung (4), lösbar miteinander verbunden sind.

11. Trokar nach Anspruch 10, wobei der Trokarkopf (1) mit
i) mindestens einem gasdichten Ventil (12) versehen ist; und/oder
ii) mindestens einen Einlass und/oder Auslass für Gas(e) und/oder Flüssigkeit(en) aufweist.

12. Trokar nach Anspruch 10 oder 11, wobei der Trokar (1,2) eine Einrichtung (13) zum Befestigen des Trokars (1,2), vorzugsweise an einem Halter oder Manipulator, aufweist.

13. Obturator mit einem Trokarrohr (2) bzw. einem Trokar (1,2) nach mindestens einem der entsprechenden vorstehenden Ansprüche, wobei zumindest ein proximaler Abschnitt (32) eines Schaftes des Obturators (3) aus flexiblem und vorzugsweise zumindest wenig kompressiblem Material hergestellt ist, so dass er sich beim Einschieben in das Trokarrohr (2) der Krümmung des proximalen Abschnitts (21) des Trokarrohrs anpassen kann und sich dabei bevorzugt wenig stauchen lässt.

14. Obturator nach Anspruch 13, wobei der Obturator (3) eine Spitze (31) aufweist, die derart konifiziert ist, dass sie der Konifizierung des freien Endes (24) des proximalen Abschnitts (21) des Trokarrohrs zum besseren Perforieren der Darmwand im Wesentlichen entspricht.

15. Obturator nach Anspruch 14, wobei die Spitze (31) des Obturators (3) einen maximalen Außendurchmesser von etwa 2-21 mm, bevorzugt von etwa 11-19 mm, weiter bevorzugt von etwa 13-17 mm, weiter bevorzugt von etwa 14-16 mm und weiter bevorzugt von etwa 15 mm aufweist.

16. Obturator nach mindestens einem der Ansprüche 14-15, wobei ein hinter der Spitze (31) des Obturators (3) angeordneter Schaft (32) des Obturators einen maximalen Außendurchmesser von etwa 6-19 mm, bevorzugt von etwa 8-17 mm, weiter bevorzugt von etwa 10-15 mm, weiter bevorzugt von etwa 11-14 mm und weiter bevorzugt von etwa 12 mm aufweist.

17. Obturator nach mindestens einem der Ansprüche 13-16, wobei der Obturator (3) einen im Wesentlichen entlang der axialen Richtung des Obturators ausgebildeten Führungskanal (83) aufweist, um den Obturator entlang eines Führungsdrahtes zu führen.

18. System mit einem Trokar nach mindestens einem der Ansprüche 10-12 und einem Obturator (3) nach mindestens einem der Ansprüche 13-15, wobei der Trokar (1,2), also das Trokarrohr (2) und der Trokarkopf (1) und die Spitze des vollständig eingeführten Obturators (3) eine Gesamtlänge von etwa 280-420 mm aufweisen.

19. System nach Anspruch 18, wobei der Obturator (3) mittels einer Rückhalteeinrichtung (70) am Trokar angebracht ist, die den Obturator in eine zurückgezogene Position vorspannt, damit die Spitze (31) des Obturators in einem geschützten Bereich innerhalb des Trokars liegt,
wobei die Rückhalteeinrichtung (70) einen Federmechanismus mit mindestens einer Feder (71) zum Vorspannen aufweist.

20. Set mit einem Trokarrohr (2) nach einem der Ansprüche 1 bis 9 und mindestens einem Element aus der Gruppe Trokar (1,2), Obturator (3) und Rektoskop (5).

## Claims

1. A trocar tube for use in the transluminal endoscopic surgery via the rectum of a human, comprising a distal portion (22) to be positioned at least in the region of or outside the rectum and a proximal portion (21) to be positioned within the human, wherein at least the proximal portion (21) comprises at least partially an essentially non-deformable curvature,
wherein the trocar tube (2) has a straight portion and the trocar tube (2) has an entire length of about 230-400 mm, **characterized in that**
the proximal portion (21):
(i) has an absolute total deflection towards one side of about 15-18 mm and
(ii) the maximum angle between a longitudinal axis of the straight portion of the trocar tube (2) and the longitudinal axis of the most bent proximal portion (21) is about 20-60°.

2. A trocar tube for use in the transluminal endoscopic surgery via the rectum of a human, comprising a distal portion (22) to be positioned at least in the region of or outside the rectum and a proximal portion (21) to be positioned within the human, wherein at least the proximal portion (21) comprises at least partially an essentially non-deformable curvature,
wherein the trocar tube (2) has an entire length of about 230-400 mm, **characterized in that**
the distal portion (22 of the trocar tube (2) is straight and has a length of 180-300 mm, and
the maximum angle between a longitudinal axis of the distal straight portion (22) of the trocar tube (2) and the longitudinal axis of the most bent proximal portion (21) is about 20-50°.

3. The trocar tube according to claim 1 or 2, wherein the entire trocar tube (2) is essentially non-deformable.

4. The trocar tube according to at least one of the preceding claims, wherein the curvature of the proximal portion (21) is essentially constant and has a curvature radius of a middle axis of the proximal portion (21) of about 45-75 mm, preferably of about 50-70 mm, further preferably of about 55-65 mm and further preferably of about 60 mm.

5. The trocar tube according to at least one of the preceding claims, wherein the trocar tube (2) has an inner diameter of about 2-22 mm, preferably of about 10-20 mm, further preferably of about 14-18 mm, further preferably of about 15-17 mm and further preferably of about 16 mm, over an at least substantial part of its entire length.

6. The trocar tube according to at least one of the preceding claims, wherein the trocar tube (2) has a wall thickness of about 0.2-2 mm, preferably of about 0.8-1.2 mm, further preferably of about 0.9-1.1 mm, further preferably of about 1 mm, over an at least substantial part of its entire length.

7. The trocar tube according to claim 1 or one of claims 3 to 6 as far as dependent on claim 1, wherein the effective length of the straight section of the trocar tube (2) is about 180-300 mm, preferably about 200-280 mm, further preferably about 220-260 mm, further preferably abut 232-252 mm, further preferably about 238-247 mm and further preferably about 242 mm.

8. The trocar tube according to at least one of the preceding claims, wherein the trocar tube (2) externally has markings for the purpose of providing readings of the radial orientation and/or its inserted length.

9. The trocar tube according to at least one of the preceding claims, wherein the trocar tube (2) preferably externally has at least one working channel.

10. A trocar comprising a trocar tube (2) according to at least one of the preceding claims and comprising a trocar head (1), wherein the trocar tube (2) and the trocar head (1) are connected to each other in a liquid-tight and/or gas-tight manner preferably by providing a seal (4) in between.

11. The trocar according to claim 10, wherein the trocar head (1)
(i) is provided with a gas-tight valve (12); and/or
(ii) comprises at least one inlet and/or outlet for gas(es) and/or liquid(s).

12. The trocar according to claim 10 or 11, wherein the trocar (1, 2) comprises a means (13) for fixing the trocar (1, 2), preferably to a holder or manipulator.

13. An obturator comprising a trocar tube (2) and/or a trocar (1, 2) according to at least one of the respective preceding claims, wherein at least one proximal portion (32) of a shaft of the obturator (3) is made of a flexible and preferably at least little compressible material so that it can adapt to the curvature of the proximal portion (21) of the trocar tube (2) when being introduced into the trocar tube and preferably undergoes almost no upsetting deformation during this operation.

14. The obturator according to claim 13, wherein the obturator (3) comprises a tip (31) which is tapered such that it essentially corresponds to the taper of the free end (24) of the proximal portion (21) of the trocar tube in order to better pierce the intestinal wall.

15. The obturator according to claim 14, wherein the tip (31) of the obturator (3) has a maximum outer diameter of about 2-21 mm, preferably of about 11-19 mm, further preferably of about 13-17 mm, further preferably of about 14-16 mm and further preferably of about 15 mm.

16. The obturator according to at least one of claims 14-15, wherein a shaft (32) of the obturator (3) arranged behind the tip (31) of the obturator has a maximum outer diameter of about 6-19 mm, preferably of about 8-17 mm, further preferably of about 10-15 mm, further preferably of about 11-14 mm and further preferably of about 12 mm.

17. The obturator according to at least one of claims 13-16, wherein the obturator (3) comprises a guide channel (83) configured essentially along the axial direction of the obturator in order to guide the obturator along a guide wire.

18. A system comprising a trocar according to at least one of claims 10-12 and an obturator (3) according to at least one of claims 13-15, wherein the trocar (1, 2), i.e. the trocar tube (2) and the trocar head (1), and the tip of the completely introduced obturator (3), has an entire length of about 280-420 mm.

19. The system according to claim 18, wherein the obturator (3) is attached to the trocar by means of a retaining means (70) which preloads the obturator into a withdrawn position in order that the tip (31) of the obturator rests in a protected region within the trocar, wherein the retaining means (70) comprises a spring mechanism having at least one spring (71) for preloading.

20. A set comprising a trocar tube (2) according to any one of claims 1 to 9 and at least one element of the group of a trocar (1, 2), an obturator (3) and a rectoscope (5).

## Revendications

1. Canule de trocart pour usage en chirurgie endoscopique transluminale via le rectum d'un patient, avec une section distale (22) pour le positionnement au moins dans la zone ou à l'extérieur du rectum et une section proximale (21) pour le positionnement à l'intérieur du patient, dans laquelle au moins la section proximale (21) présente au moins partiellement une courbure sensiblement non déformable,
dans laquelle la canule de trocart (2) présente une section droite et la canule de trocart (2) a une longueur totale d'environ 230 à 400 mm,
**caractérisé en ce que** la section proximale (21) :
i) présente une déviation totale absolue vers un côté d'environ 15 à 18 mm et,
ii) l'angle maximal entre un axe longitudinal de la section droite de la canule de trocart et l'axe longitudinal de la section proximale la plus largement courbée s'élève à environ 20 à 60°.

2. Canule de trocart pour usage en chirurgie endoscopique transluminale via le rectum d'un patient, avec une section distale (22) pour le positionnement au moins dans la zone ou à l'extérieur du rectum et une section proximale (21) pour le positionnement à l'intérieur du patient, dans laquelle au moins la section proximale (21) présente au moins partiellement une courbure sensiblement non déformable,
dans laquelle la canule de trocart (2) a une longueur totale d'environ 230 à 400 mm,
**caractérisé en ce que**
la section distale (22) de la canule de trocart (2) est droite et présente une longueur de 180 à 300 mm et
l'angle maximal entre un axe longitudinal de la section distale droite (22) de la canule de trocart et l'axe longitudinal de la section proximale (21) la plus largement courbée s'élève à environ 20 à 50°.

3. Canule de trocart selon la revendication 1 ou 2, dans laquelle la canule de trocart (2) complète est sensiblement indéformable.

4. Canule de trocart selon au moins l'une quelconque des revendications précédentes, dans laquelle la courbure de la section proximale (21) est sensiblement constante et présente un rayon de courbure d'un axe central de la section proximale (21) d'environ 45 à 75 mm, de préférence d'environ 50 à 70 mm, mieux encore d'environ 55 à 65 mm et bien mieux encore d'environ 60 mm.

5. Canule de trocart selon au moins l'une quelconque des revendications précédentes, dans laquelle la canule de trocart (2) présente sur une partie au moins sensible de la longueur totale un diamètre interne d'environ 2 à 22 mm, de préférence d'environ 10 à 20 mm, mieux encore d'environ 14 à 18 mm, bien mieux encore d'environ 15 à 17 mm et nettement mieux encore d'environ 16 mm.

6. Canule de trocart selon au moins l'une quelconque des revendications précédentes, dans laquelle la canule de trocart (2) présente sur une partie au moins sensible de la longueur totale une épaisseur de paroi d'environ 0,2 à 2 mm, de préférence d'environ 0,8 à 1,2 mm, mieux encore d'environ 0,9 à 1,1 mm, bien mieux encore d'environ 1 mm.

7. Canule de trocart selon la revendication 1 ou l'une quelconque des revendications 3 à 6, pour autant qu'elle dépend de la revendication 1, dans laquelle la longueur efficace de la section droite de la canule de trocart s'élève à environ 180 à 300 mm, de préférence environ 200 à 280 mm, mieux encore environ 220 à 260 mm, bien mieux encore environ 232 à 252 mm, plus nettement environ 238 à 247 mm et plus nettement encore environ 242 mm.

8. Canule de trocart selon au moins l'une quelconque des revendications précédentes, dans laquelle la canule de trocart (2) présente à l'extérieur des marques pour lire l'orientation radiale et/ou la longueur introduite.

9. Canule de trocart selon au moins l'une quelconque des revendications précédentes, dans laquelle la canule de trocart (2) présente encore de préférence à l'extérieur au moins un canal de travail.

10. Trocart avec une canule de trocart (2) selon au moins l'une quelconque des revendications précédentes et une tête de trocart (1) dans lequel la canule de trocart (2) et la tête de trocart (1) sont reliés l'un à l'autre de manière amovible, de façon étanche aux liquides ou aux gaz, de préférence avec interposition d'un joint d'étanchéité (4).

11. Trocart selon la revendication 10, dans lequel la tête de trocart (1)
i) est pourvue d'au moins une soupape étanche aux gaz (12) ; et/ou
ii) présente au moins une entrée et/ou une sortie pour le(s) gaz et/ou le(s) liquide(s).

12. Trocart selon la revendication 10 ou 11, dans lequel le trocart (1, 2) présente un dispositif (13) pour fixer le trocart (1, 2), de préférence sur un support ou un manipulateur.

13. Obturateur avec une canule de trocart (2) ou un trocart (1, 2) selon au moins l'une quelconque des revendications précédentes correspondantes, dans lequel au moins une section proximale (32) d'une tige de l'obturateur (3) est fabriquée dans un matériau souple et, de préférence, au moins peu compressible de sorte qu'il puisse s'adapter à la courbure de la section proximale (21) de la canule de trocart lors de l'insertion dans la canule de trocart (2) et se laisse de préférence peu comprimer.

14. Obturateur selon la revendication 13, dans lequel l'obturateur (3) présente une pointe (31) qui est taillée en cône de telle sorte qu'elle corresponde sensiblement à la forme en cône de l'extrémité libre (24) de la section proximale (21) de la canule de trocart pour une meilleure perforation de la paroi intestinale.

15. Obturateur selon la revendication 14, dans lequel la pointe (31) de l'obturateur (3) présente un diamètre extérieur maximal d'environ 2 à 21 mm, de préférence d'environ 11 à 19 mm, mieux encore d'environ 13 à 17 mm, bien mieux encore d'environ 14 à 16 mm et nettement mieux encore d'environ 15 mm.

16. Obturateur selon au moins l'une quelconque des revendications 14 et 15, dans lequel une tige (32) de l'obturateur agencée derrière la pointe (31) de l'obturateur (3) présente un diamètre extérieur maximal d'environ 6 à 19 mm, de préférence d'environ 8 à 17 mm, mieux encore d'environ 10 à 15 mm, bien mieux encore d'environ 11 à 14 mm et nettement mieux encore d'environ 12 mm.

17. Obturateur selon au moins l'une quelconque des revendications 13 à 16, dans lequel l'obturateur (3) présente un canal de guidage (83) formé sensiblement le long de la direction axiale de l'obturateur pour guider l'obturateur le long d'un fil de guidage.

18. Système avec un trocart selon au moins l'une quelconque des revendications 10 à 12 et un obturateur (3) selon au moins l'une quelconque des revendications 13 à 15, dans lequel le trocart (1, 2), c'est-à-dire la canule de trocart (2) et la tête de trocart (1), et la pointe de l'obturateur (3) complètement introduit présentent une longueur totale d'environ 280 à 420 mm.

19. Système selon la revendication 18, dans lequel l'obturateur (3) est appliqué contre le trocart au moyen d'un dispositif de retenue (70) qui précontraint l'obturateur dans une position de retrait afin que la pointe (31) de l'obturateur se trouve dans une zone protégée à l'intérieur du trocart,
dans lequel le dispositif de retenue (70) présente un mécanisme à ressort avec au moins un ressort (71) pour la précontrainte.

20. Ensemble avec une canule de trocart (2) selon l'une quelconque des revendications 1 à 9 et au moins un élément du groupe formé par le trocart (1,2) l'obturateur (3) et le rectoscope (5).
